# EUROPEAN PATENT SPECIFICATION

(11) **EP 2 528 600 B1**
(45) Date of publication and mention of the grant of the patent: **29.06.2016**
(21) Application number: 11706368.5
(22) Date of filing: 28.01.2011
(51) Int. Cl.: A61K 31/381, A61K 31/46, A61K 31/575, A61K 9/00, A61P 11/00, A61P 11/06

(54) **DRY POWDER PHARMACEUTICAL COMPOSITION COMPRISING TIOTROPIUM AND FLUTICASONE**
PHARMAZEUTISCHE TROCKENPULVERZUSAMMENSETZUNG MIT TIOTROPIUM UND FLUTICASON
COMPOSITION PHARMACEUTIQUE DE POUDRE SÈCHE COMPRENANT LE TIOTROPIUM ET LE FLUTICASONE

(30) Priority: 02.02.2010 TR 201000731; 28.01.2010 TR 201000624
(43) Date of publication of application: 05.12.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000015
(87) International publication number: WO 2011/093813

(56) References cited:
- EP-A1- 0 418 716
- WO-A1-01/78739
- WO-A1-2004/110404
- WO-A1-2005/053648
- US-A- 4 335 121
- US-A1- 2002 183 292
- CAZZOLA ET AL: "A pilot study to assess the effects of combining fluticasone propionate/salmeterol and tiotropium on the airflow obstruction of patients with severe-to-very severe COPD", PULMORNARY PHARMACOLOGY & THERAPEUTICS, ACADEMIC PRESS, GB, vol. 20, no. 5, 20 July 2007 (2007-07-20), pages 556-561, XP022162533, ISSN: 1094-5539, DOI: DOI:10.1016/J.PUPT.2006.06.001

## Description

The present invention relates to a pharmaceutical composition in dry powder form comprising tiotropium and fluticasone and/or their pharmaceutically acceptable derivatives as active agents so as to be used in the treatment of respiratory tract diseases especially in asthma and chronic obstructive pulmonary disease (COPD), and the delivery of this pharmaceutical composition.

Airways, in other words bronchia, are the channels which function to distribute the inhaled air into the lung tissues. In the case of respiratory diseases such as asthma or chronic obstructive pulmonary disease (COPD), stimulants such as allergen, infection, good and bad smell, smoke, genetic factors and exercise cause contractions in the airway muscles (bronchoconstruction) and/or excessive secretion in glands and results in contractions in the airway. Hence, respiration gets more difficult as the inhaled air cannot be exhaled.

Corticosteroids which are used in the treatment of asthma and COPD are synthetic and strong anti-inflammatory drugs that are similar to natural corticosteroid hormones produced by adrenal glands. They prevent both the transcription of the inflammatory gene and the activation of the anti-inflammatory gene. They are known to be the most effective drug in the treatment of asthma.

Fluticasone, which is the one of the molecules in the group of "corticosteroids" and has the chemical name S-(fluoromethyl) (6S,8S,9R,10S,11S,13S,14S,16R,17R)-6,9-difluoro-11,17-dihydroxy 10,13,16-trimethyl-3-oxo-6,7,8,11,12,14,15,16-octahydrocyclopenta[a]phenanthrene-17-carbothioate, was first disclosed in the patent numbered US4335121.

Corticosteroids are not preferred in acute asthma crises as they do not have rapid onset of action. Since inhaled corticosteroids may prevent growth in children, they are advised to be used in the lowest possible amount. Long-term use of corticosteroids may cause cataract and glaucoma. In addition, they may cause some serious side-effects such as osteoporosis, high cholesterol, edema, encepholalgia, weight gain, insomnia and some skin problems.

Anticholinergics are the other active agents which are utilized in the treatment of respiratory disorders. Anticholinergics affect muscarinic receptors and prevent the obliteration in the bronchia due to excessive secretion. These drugs are categorized into two groups as long-acting and short-acting. Short-acting anticholinergics including ipratropium bromide and oxitropium bromide have an onset of action of 15 minutes and their duration of action is 6-8 hours. The long- acting anticholinergic agent is tiotropium. Tiotropium, having the chemical name (1R, 2R, 4S, 5S, 7S)-7-[(hydroxidi-2-thienylacetyl)oxy]-9,9-dimethyl-3-oxa-9-azoniatricyclo [3.3.1.0^{2,4}] nonane, was first described in the patent application EP0418716. Tiotropium's onset of action is 20 minutes but its duration of action is 24 hours. Thus, it is enough to take it once a day. Xerostomia, blurred vision, glaucoma and dry cough are its side-effects.

The use of combination drugs in the treatment of respiratory diseases such as asthma and COPD is very effective particularly in decreasing asthma attacks. It is possible that the severity or occurrence possibility of the abovementioned side effects decreases as the active substances that are used in combinations are more effective at lower doses compared to the active substances used alone. In this context the disclosure of WO2004110404 is relevant.

However, decreasing the side effects that arise from the active agents is not sufficient to provide effective treatment for respiratory diseases. Medicaments used in the formulations should be selected in a way to give the best combination and furthermore they should be in the most stable form. Moreover, the compositions comprising them should be formulated in such a way that the composition is stable and also it reaches to the target area in the most efficient way.

The inventor has found that therapeutic benefits are obtained through the use of the combination comprising tiotropium bromide with water content less than or equal to 2.5%, and fluticasone propionate together in dry powder form for simultaneous or sequential administration in the prevention or treatment of respiratory diseases.

It was seen that a combination comprising tiotropium bromide with water content less than or equal to 2.5%, and fluticasone propionate in dry powder form provides the most stable and therapeutically beneficial combination for simultaneous or sequential administration in the prevention or treatment of respiratory diseases.

Furthermore, the amount of the active agents used in the composition is carefully adjusted in order to prevent side effects that might arise from these agents and it was seen that the adhesive force between the particles forming the composition is reduced and hence the amount of inhaled particles and efficacy of the formulation increases in addition to observing minimum side effects, when tiotropium bromide with water content less than or equal to 2.5% and fluticasone propionate present in the composition is used in an amount that the ratio of tiotropium bromide with water content less than or equal to 2.5% to fluticasone propionate is in the range of 1:3 to 1:50 by weight.

In order to ensure effective absorption of the active agents into the lung tissue, the particle size of the agents should be adjusted. Although large particle size provides ease in the manufacture of the dry powder, it may accumulate in throat and lead to insufficient intake of the medicament. Very fine particles, on the other hand, may reach the lungs. However, they might not have a good flow property which, in turn, causes problems in providing dose accuracy. To prevent these problems, the active agents should have an optimum average particle size. The inventors have found that the combination of tiotropium bromide with a water content less than or equal to 2.5% and fluticasone propionate wherein the mean particle size of the active agents is in the range of 1.5 to 4.5 µm reaches the lungs effectively and no problems related to flow properties of the dry powder are observed.

The term "mean particle size" refers to particles wherein the size of 50% of the total number of particles is less than the average particle size.

In one aspect, the present invention is related to dry powder formulations comprising a combination of tiotropium bromide with a water content less than or equal to 2.5% and fluticasone propionate in dry powder form wherein tiotropium bromide with a water content less than or equal to 2.5% and fluticasone propionate is present in the composition in an amount that the ratio of tiotropium bromide with water content less than or equal to 2.5% to fluticasone propionate is in the range of 1:3 to 1:50 by weight and wherein the mean particle size of the active agents (i.e. tiotropium bromide with water content less than or equal to 2.5% and fluticasone propionate) is in the range of 1.5 to 4.5 µm.

According to the present invention, the drug comprising tiotropium bromide with a water content less than or equal to 2.5% and fluticasone propionate may also contain effective amounts of excipients and/or additional agents apart from active agents.

According to the present invention, the dry powder formulation comprising tiotropium bromide with a water content less than or equal to 2.5% and fluticasone propionate is transmitted to the patient in dry powder form. Said dry powder formulations also contain some physiologically acceptable excipients along with the active agent. These excipients can be monosaccharides (glucose, etc.), disaccharides (lactose, saccharose, maltose, etc.), oligosaccharides and polysaccharides (dextran, etc.), polyalcohols (sorbitol, mannitol, xylitol, etc.), salts (sodium chloride, calcium carbonate, etc.) or a mixture thereof.

The inventors have found that in the compositions pertaining to the present invention, in other words in the compositions comprising tiotropium bromide with water content less than or equal to 2.5% and fluticasone propionate in dry powder form wherein the mean particle size of tiotropium bromide with water content less than or equal to 2.5% and fluticasone propionate is in the range of 1.5 to 4.5 µm and wherein tiotropium bromide with water content less than or equal to 2.5% and fluticasone propionate is present in the composition in an amount that the ratio of tiotropium bromide with water content less than or equal to 2.5% to fluticasone propionate is in the range of 1:3 to 1:50 by weight, using lactose as the one and only carrier provides optimum homogeneity and flow properties to the dry powder and therefore, dose accuracy is maintained.

In other words, it is another aspect of the present invention that lactose is used as the one and only carrier in dry powder formulations comprising combinations of tiotropium bromide with a water content less than or equal to 2.5% and fluticasone propionate in dry powder form.

It was also seen that the mean particle size of the lactose plays an important role in the effective delivery of the medicament to the target area, i.e. lungs, in the compositions pertaining to the present invention. It was found that in the case that lactose, which has a mean particle size less than or equal to 100 µm, is used in compositions comprising tiotropium bromide with water content less than or equal to 2.5% and fluticasone propionate in dry powder form wherein mean particle size of tiotropium bromide with water content less than or equal to 2.5% and fluticasone propionate is in the range of 1.5 to 4.5 µm and wherein said active agents are present in the composition with the ratio of 1:3 to 1:50 respectively, the adhesive forces present between the lactose particles and the active agents having the mean particle size in the range of 1.5 to 4.5 µm are minimized and thus, an effective inhalation of the active agents takes place.

In another aspect, the present invention provides a composition comprising tiotropium bromide with water content less than or equal to 2.5% and fluticasone propionate in dry powder form wherein;
- mean particle size of tiotropium bromide with water content less than or equal to 2.5% and fluticasone propionate is in the range of 1.5 to 4.5 µm and
- said active agents are present in the composition with the ratio of 1:3 to 1:50 respectively and
- lactose which has a mean particle size less than 100 µm is used as carrier.

Lactose that has a mean particle size less than 100 µm is preferably used as a mixture of particles which have two different mean particle sizes. Accordingly, lactose that has a mean particle size less than 100 µm can be present as a mixture of particles having a mean particle size less than 10 µm (fine) and particles having a mean particle size in the range of 10 µm to 100 µm (coarse). The inventors have observed that when lactose which has two different mean particle sizes is used, the adhesive force between the active agents and lactose is even less.

The fine lactose particles have a mean particle size less than 10 µm, preferably between 2 µm and 8µm and the coarse lactose particles have a mean particle size less than 100 µm, preferably between 30 µm and 80 µm.

The ratio between the fine lactose particles which has a mean particle size less than 10 µm and the coarse lactose particles which has a mean particle size in the range of 10 µm to 100 µm is in the range of 20:80% and 40:60% by weight.

According to the present invention, the amount of the pharmaceutically acceptable carrier is preferably in the range of 0-50 mg.

The medicament combination of the present invention is in dry powder form and it is inhaled via dry powder inhalers. Accordingly, the medicament can be inhaled via an inhalator including a reservoir containing dry powder, a blister pack in which many blisters are placed in an order or a capsule pack. Among these, the inhalers that enable intake of a single dose in an accurate manner were found to give the best results. This condition is provided by the dosage forms wherein each dose of the medicament is stored in a single unit of dosage form. Accordingly, the medicament combination according to the present invention is stored in blister packs consisting of blisters or capsules and is inhaled via dry powder inhalation devices where blisters or capsules are used.

The inventor has surprisingly found that administering the dry power drug comprising tiotropium bromide with water content less than or equal to 2.5% and fluticasone propionate wherein;
- the mean particle size of tiotropium bromide with water content less than or equal to 2.5% and fluticasone propionate is in the range of 1.5 to 4.5 µm and
- said active agents are present in the composition with the ratio of 1:3 to 1:50 respectively and
lactose that has mean a particle size less than 100 µm is used as carrier as stored in a blister pack or capsule via a dry powder inhaler in order to prevent and treat respiratory diseases provides the transmission of the exact and the effective dose to the target area and elimination and/or alleviation of the known side effects of the therapeutic agents. Hence, maximum therapeutic benefit is provided due to the efficacy of the transmission method.

According to another aspect, the present invention provides a dry powder drug comprising the combination of tiotropium bromide with water content less than or equal to 2.5% and fluticasone propionate wherein;
- the mean particle size of tiotropium bromide with water content less than or equal to 2.5% and fluticasone propionate is in the range of 1.5 to 4.5 µm and
- said active agents are present in the composition with the ratio of 1:3 to 1:50 respectively, and
lactose that has a mean particle size less than 100 µm is used as carrier which realizes a simultaneous inhalation from a blister pack or a capsule that can guarantee a single dose intake at once in order to achieve dose sufficiency.

According to another aspect, the present invention provides inhalation of the dry powder drug comprising the combination of tiotropium bromide with water content less than or equal to 2.5% and fluticasone propionate wherein;
- mean particle size of tiotropium bromide with water content less than or equal to 2.5% and fluticasone propionate is in the range of 1.5 to 4.5 µm and
- said active agents are present in the composition with the ratio of 1:3 to 1:50 respectively and
- lactose that has a mean particle size less than 100 µm is used as carrier via a simple, low cost, trustable dry powder inhaler.

According to another aspect, the present invention provides a drug that provides to realize the use of a composition comprising tiotropium bromide with water content less than or equal to 2.5% and fluticasone propionate wherein;
- mean particle size of tiotropium bromide with water content less than or equal to 2.5% and fluticasone propionate is in the range of 1.5 to 4.5 µm and
- said active agents are present in the composition with the ratio of 1:3 to 1:50 respectively and
lactose that has a mean particle size less than 100 µm is used as carrier as stored together in peelable blister or pierceable capsule packs in the treatment of respiratory diseases such as asthma and COPD.

The inventors have found that by storing the dry powder medicament pertaining to the present invention in blister packs comprising blisters and capsules packs which have the specified properties pertaining to the present invention,
- equal dose intake is provided in each use as the dry powder formulation is filled into the blisters or capsules with a good dosage accuracy in the factory after the manufacture,
- the drug is transmitted to the target area, which is the lungs, without absorbing moisture as the blister or capsule pertaining to the present invention is peeled/torn/pierced immediately before use,
- inhalation of inadequate or excessive dose is prevented and inhalation of a unit dose is ensured as it is single dose,
- the dry powder formulation pertaining to the present invention is effectively transmitted to the lungs from the blisters or capsules pertaining to the present invention via a dry powder inhaler, and
- devices containing blisters or capsules provide ease of use to the patients as they are small in size resulting from the fact that they work with simple mechanical components compared to other devices.

The present invention relates to a delivery method of a drug composition containing a composition comprising tiotropium bromide with water content less than or equal to 2.5% and fluticasone propionate wherein;
- the mean particle size of tiotropium bromide with water content less than or equal to 2.5% and fluticasone propionate is in the range of 1.5 to 4.5 µm and
- said active agents are present in the composition with the ratio of 1:3 to 1:50 respectively and
- lactose that has a mean particle size less than 100 µm is used as carrier,
which is particularly used in the treatment of people suffering from respiratory diseases such as asthma, COPD and allergic rhinitis, as stored in a blister or capsule package via a dry powder inhaler.

If capsules are used for packaging the dry powder medicament according to the invention, the piercing components existing in the device to prepare the dry powder drug carried in a capsule for inhalation pierce the capsule when the device is triggered and the dry powder drug kept in the capsule gets ready for inhalation. After the inhalation is completed, the empty capsule is ejected from the device and a new capsule is placed immediately before the following inhalation takes place.

The capsule package, which is preferred to be used in the scope of the present invention, consists of two intertwining parts.

In the present invention, the inventors have found that the ideal inhalation conditions are achieved when the volume of the capsule, which contains the dry powder drug comprising tiotropium bromide with water content less than or equal to 2.5% and fluticasone propionate wherein;
- the mean particle size of tiotropium bromide with water content less than or equal to 2.5% and fluticasone propionate is in the range of 1.5 to 4.5 µm and
- said active agents are present in the composition with the ratio of 1:3 to 1:50 respectively, and
lactose which has a mean particle size less than 100 µm is used as carrier, is in the range of 0.1 to 0.52 ml, preferably in the range of 0.1 to 0.45 ml, more preferably in the range of 0.15 to 0.42 ml.

Accordingly, the capsule that is used to store and transmit the combination comprising tiotropium bromide with water content less than or equal to 2.5% and fluticasone propionate wherein;
- the mean particle size of tiotropium bromide with water content less than or equal to 2.5% and fluticasone propionate is in the range of 1.5 to 4.5 µm and
- said active agents are present in the composition with the ratio of 1:3 to 1:50 respectively and
- lactose which has a mean particle size less than 100 µm is used as carrier
is characterized in having a cavity volume in the range of 0.1 to 0.52 ml, preferably 0.1 to 0.45 ml, more preferably in the range of 0.15 to 0.42 ml.

According to another aspect, the capsule package which has high protection against moisture and other negative external factors has a moisture rate between 10%-20%, preferably 15%-20%. In the case that the capsules having this characteristic are used, both the active composition is protected from external factors and the probability of moisture arising from the capsule's own structure are prevented. Thus, the most effective transmission to the patient is enabled by preventing the agglomeration of the dry powder.

The capsule, which is preferred to be used in scope of the present invention, can be made of a substance chosen from a group including gelatine, chitosan, starch and/or starch derivatives, cellulose and/or cellulose derivatives or synthetic polymers as well as consisting intertwined upper and lower parts. These upper and lower parts of said capsule can be produced of identical or different materials.

In the case that the capsule used in the present invention is made of cellulose or its derivatives, the capsule material can be selected from, but not limited to, a group comprising hydroxypropyl cellulose, hydroxypropylmethyl cellulose, methyl cellulose, hydroxymethyl cellulose, hydroxyethyl cellulose.

In the case that the capsule used in the present invention is synthetic polymer, the capsule material can be selected from, but not limited to, a group including polyethylene, polyetheleneteraphtalate, polycarbonate or polypropylene.

In the case that the capsule material used in the present invention is gelatine, additional agents such as polyethylene glycol, sorbitol, glycerol, propylene glycol, polyethylene oxide - polypropylene oxide block copolymers and/or other polyalcohols or polyethers at different molecular weights can be added into it.

According to another aspect, the present invention is characterized in that the capsule cavity used to provide an effective inhalation of the dry powder medicament is filled up to 0.01% to 25% of its total volume, preferably 0.1 to 20% of its total volume, more preferably 0.5 to 17% of its total volume.

According to this, the present invention comprises dry powder formulations containing tiotropium and fluticasone propionate and/or pharmaceutically acceptable derivatives and their administration to patients from a capsule having a total cavity volume in the range of 0.1 to 0.52 ml wherein the capsule cavity is filled up to 0.01% to 25% of the total volume, via a dry powder inhaler.

In addition, the capsule pack pertaining to the present invention can be in any color or shape as long as it has the properties described above.

The capsule or the blister pack, which comprises the formulation pertaining to the present invention, can be used with any dry powder inhalation device, for example with devices as described in the patent applications numbered TR2008/03522, TR2008/03523, TR2010/03091, TR2010/04311.

In another aspect, blister packages can be used to pack the dry powder medicament according to the invention. Blister packages can be a) torn b) pierced or c) peeled to be opened according to the structure of the device and the blister. As a result of the studies and experiments that they conducted, the inventors have found that it is difficult to use dry powder inhalers which contain pierceable or tearable blister packs for patients as these devices require additional components which increase the size, volume and the complexity of the device. In addition, the amount of uninhaled dry powder formulation remaining in the blister cavity increases due to the roughness resulting from being torn and therefore, the rate of utilization from the present dry powder formulation decreases in tearable blisters. In contrast, the amount of dry powder remaining in the blister cavity decreases as said dry powder combination is transmitted much more effectively with a high discharge capacity in the case that peelable blisters are used.

The dry powder medicament pertaining to the present invention can be carried in peelable blister packages. Blister packs are comprised of orderly placed blisters each of which contains minimally one dose of the dry powder drug. When the device is triggered, the blister pack or one of the blisters in the pack is peeled and the drug in dry powder form is prepared for inhalation.

According to the present invention, the cavity volume of the blisters that are arranged side by side in a certain order on the blister pack which provides to carry and store the drug in dry powder from is 17 to 30 mm³, preferably 18 to 23 mm³, most preferably 19 to 21 mm³

The cavity volume of the blisters pertaining to the present invention, which provide to transmit and store the dry powder drug containing tiotropium bromide with water content less than or equal to 2.5% and fluticasone propionate wherein;
- the mean particle size of tiotropium bromide with water content less than or equal to 2.5% and fluticasone propionate is in the range of 1.5 to 4.5 µm and
- said active agents are present in the composition with the ratio of 1:3 to 1:50 respectively, and
lactose, which has a mean particle size less than 100 µm, is used as carrier is in the range of 17-30 mm³, preferably in the range of 18-23 mm³, most preferably in the range of 19-21 mm³ and each blister cavity having the volume described above is filled up to 25-100 %, preferably up to 70-100 %, most preferably up to 90-100 % of said volume in order to meet the specified needs for an effective inhalation.

The lid and the base sheets of said blister pack constituted by the blisters having the specified properties, in which the drug in dry powder form pertaining to the present invention is stored, are closed very tightly by any suitable method to provide impermeability.

According to the present invention, the lid and the base sheets constituting the blister package consist of several layers. Polymeric layers, aluminum foil and preferably Aclar® fluoropoylmer film are among the layers that form the lid and the base sheet.

Aclar® fluoropolymer film is a polymeric film which is used in blister packs and provides excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

In order to decrease gas and moisture permeability of the layer, preferably desiccant agents are added to the polymeric layers to preserve the stability of the dry powder formulation stored in blisters that are arranged in an order on the blister package. Silica gel, zeolite, alumina, bauxite, anhydrous calcium sulfate, activated carbon and clay which have the property of water absorption can be given as examples to desiccant agents.

As it is common to use aluminum in lid and base sheets of high protection blister packs, aluminum is used both in the lid and the base sheets of the blister pack of the present invention in order to provide high moisture and gas protection. These aluminum foils must be thick enough to provide the desired protection for the stability of the moisture sensitive dry powder formulation stored in the blister cavity. Due to this reason, the thickness of the aluminum foil that is used in the lid and the base sheets of the blister pack is chosen to be in the range of 10 to 40 µm, preferably of 15 to 30 µm.

According to the present invention, the polymeric layers in the lid and the base sheets of the blister pack mentioned in the present invention are composed of the same or different polymers. The thickness of these polymeric layers varies according to the type of the polymeric substance used and its properties. Therefore, the thickness of the polymeric layer varies in the range of 15-60 µm, preferably of 20-35 µm depending on the type of the polymer used.

The blisters which constitute the blister pack in which the dry powder drug pertaining to the present invention is stored can be in any shape as long as they have the properties described above.

Due to the electrostatic interactions between the dry powder particles and the inside layer of the blister cavity or the capsule material which is in contact with the dry powder formulation, the dry powder particles mainly adhere to said inside layer of the blister cavity or said capsule material. Since some uninhaled dry powder formulation is remained in the blister cavity or the capsule during inhalation, the sufficient amount of the dry powder formulation for an effective therapy cannot reach to the lungs. Therefore, the inside layer of the blister cavity or the capsule material that is in contact with the dry powder formulation is an antistatic material. Within the scope of the invention, the term "antistatic material" refers to a material which eliminates the buildup of static electricity. The antistatic material used herein encloses a material which is antistatic itself or which is not antistatic itself but contains antistatic agent(s).

According to the present invention, an antistatic agent is used for treatment of the layer or their surfaces in order to reduce or eliminate the buildup of static electricity generally caused by the triboelectric effect (charge generation by friction). Its role is to make the surface or the material itself slightly conductive either by being conductive itself or by absorbing moisture from the air. Therefore, some humectants can be used. The molecules of an antistatic agent often have both hydrophilic and hydrophobic areas similar to those of a surfactant. The hydrophobic side interacts with the surface of the material while the hydrophilic side interacts with the air moisture and binds the water molecules.

Antistatic agents are basically classified into two groups: internal antistatic agents and external antistatic agents. Internal antistatic agents are designed to be mixed directly into the material whereas external antistatic agents are applied to the surface.

Antistatic agents used within the scope of the invention are selected from a group comprising long-chain aliphatic amines (optionally ethoxylated) and amides, glycerol monostearate (GMS), saturated fatty acids, (poly)unsaturated fatty acids, quaternary ammonium salts (e.g., behentrimonium chloride or cocamidopropyl betaine), sulfonated organic compounds, esters of phosphoric acid, polyethylene glycol esters, or polyols and combinations thereof. In addition, some commercialy available antistatic additives such as OnCap™, Larostat ®, Entira™, Nourymix®, can be used as antistatic agents. It is also possible to use conductive polymers like PEDOT:PSS and conducting polymer nanofibers, particularly polyaniline nanofibers.

Processing conditions, polymer base, relative humidity of the environment, material thickness and active agents used in the dry powder formulation play an important role in selecting the most appropriate antistatic agent. Also, the bloom rate (the rate of migration of the antistatic agent to the surface) varies greatly among the antistatic agent choices. The antistatic agents in the material, which is the inside layer of the blister cavity or capsule material, are present in a range from 0,1% to 5% by weight of said material.

In another aspect, the present invention provides a medicament comprising a composition of tiotropium bromide with water content less than or equal to 2.5% and fluticasone propionate wherein;
- the mean particle size of tiotropium bromide with water content less than or equal to 2.5% and fluticasone propionate is in the range of 1.5 to 4.5 µm and
- said active agents are present in the composition with the ratio of 1:3 to 1:50 respectively and
- lactose which has a mean particle size less than 100 µm is used as carrier
as stored in capsules or blisters wherein the inside layer of the blister cavity or the capsule material that is in contact with the dry powder formulation is an antistatic material.

According to the present invention, tiotropium bromide with water content less than or equal to 2.5%, which is one of the active agents of the medicament formulation comprising the active agent combination, includes its pharmaceutically acceptable solvates, polymorphs, crystal forms, amorphous forms and/or combinations thereof.

According to the present invention, fluticasone propionate, which is one of the active agents of the medicament formulation comprising the active agent combination, includes its pharmaceutically acceptable solvates, hydrates, enantiomers, racemates, free base, polymorphs, crystal forms, amorphous forms and/or combinations thereof.

According to the present invention, the amount of tiotropium bromide with water content less than or equal to 2.5% is in the range of 1 to 40µg, preferably in the range of 1 to 30 µg in the medicament formulation in dry powder form comprising the combination of tiotropium with water content less than or equal to 2.5% and fluticasone propionate and/or pharmaceutically acceptable derivatives thereof.

According to the present invention, the amount of fluticasone propionate in the range of 1 to 600µg, preferably in the range of 10 to 550 µg in the medicament formulation in dry powder form comprising the combination of tiotropium and fluticasone propionate and/or pharmaceutically acceptable derivatives thereof.

The dry powder drug comprising tiotropium bromide with water content less than or equal to 2.5% and fluticasone propionate wherein;
- the mean particle size of tiotropium bromide with water content less than or equal to 2.5% and fluticasone propionate is in the range of 1.5 to 4.5 µm and
- said active agents are present in the composition with the ratio of 1:3 to 1:50 respectively and
- lactose which has a mean particle size less than 100 µm is used as carrier
as stored in capsules or blisters wherein inside layer of said capsules or blisters is made of a material having anti electrostatic properties according to the present invention can be used in the treatment of several respiratory diseases such as asthma, chronic obstructive pulmonary disease (COPD) and allergic rhinitis. Accordingly, these respiratory diseases can be, but not limited to, allergic and non-allergic asthma at any phases, acute lung injury (ALI), acute respiratory distress syndrome (ARDS), intensifying of airways hyperactivity, bronchiectasis, chronic obstructive pulmonary, airways or lung disease including amphizem and chronic bronchitis, pneumokoniosis, aluminosis, antracosis, asbetosis, calicosis, phytilosis, ciderosis, silicosis, tabacosis, bissnosizn. This treatment can be prophylactic or symptomatic. In addition, said composition is preferably used for symptomatic treatment of asthma, COPD and allergic rhinitis.

A method for preparing the pharmaceutical composition according to the present invention comprises micronizing the tiotropium bromide with water content less than or equal to 2.5% and fluticasone propionate, preferably by air jet mill, mixing micronized tiotropium bromide with water content less than or equal to 2.5% and fluticasone propionate with lactose and blending the composition to obtain a homogeneous dry powder mixture and then filling the obtained dry powder mixture into capsules or blisters.

The pharmaceutical composition pertaining to the present invention can be explained with, but not limited to, the examples given below.

### EXAMPLE 1

The dry powder formulation which is appropriate for a gelatine capsule used in the capsule inhalator comprises 18 parts of tiotropium bromide with water content less than or equal to 2.5% which was micronized in air jet mill and has a mean particle size of 1,5 to 4.5 µm; 200 parts of fluticasone propionate which was micronized in air jet mill and has a mean particle size of 1.5 to 4.5 µm, and 4500 parts of lactose as carrier which has a mean particle size below 100µm.

Tiotropium bromide with water content less than or equal to 2.5% given in this example comprises its all pharmaceutically acceptable solvates, polymorphs, amorphous forms and crystalline forms. Fluticasone propionate given in this example comprises its all pharmaceutically acceptable solvates, hydrates and/or enantiomers, polymorphs, amorphous and crystal forms. Lactose, which is used as carrier, can optionally be added in a higher or a lower amount. The capsule described in the example is made of gelatine and it can optionally be made of chitosan, starch and/or starch derivatives, cellulose and/or cellulose derivatives or synthetic polymers. The inside layer of the blister cavity or the capsule material that is in contact with the dry powder formulation is an antistatic material. The amounts given in the table below can be replaced with the amounts given in example 1:

| Example | Amount of tiotropium active (parts) | Amount of fluticasone propionate (parts) | Amount of lactose (parts) |
|---|---|---|---|
| 2 | 11 | 200 | 4000 |
| 3 | 11 | 200 | 5000 |
| 4 | 11 | 200 | 4500 |
| 5 | 20 | 400 | 5500 |
| 6 | 20 | 400 | 5000 |
| 7 | 20 | 400 | 6000 |
| 8 | 20 | 200 | 4000 |
| 9 | 20 | 400 | 6000 |
| 10 | 11 | 200 | 5750 |
| 11 | 11 | 200 | 4750 |
| 12 | 11 | 200 | 4200 |
| 13 | 11 | 200 | 5200 |
| 14 | 11 | 400 | 5500 |
| 15 | 20 | 200 | 4600 |
| 16 | 20 | 400 | 4800 |
| 17 | 11 | 400 | 6000 |
| 18 | 11 | 400 | 6000 |
| 19 | 20 | 400 | 4750 |

### EXAMPLE 20

A dry powder formulation which is suitable to be stored in blisters so as to be used in a multiple dosing inhalator comprises 18 pieces of tiotropium bromide with water content less than or equal to 2.5% which has an average particle diameter of 1.5-4.5 µm and was micronized in air jet mill; 200 pieces of fluticasone propionate which was micronized in air jet mill, and 1000 pieces of lactose having a particle diameter of less than 100 µm as a carrier.

Tiotropium bromide with water content less than or equal to 2.5% given in this example comprises its all pharmaceutically acceptable solvates, polymorphs, amorphous forms and crystalline forms. Fluticasone propionate given in this example comprises its all pharmaceutically acceptable solvates, hydrates and/or enantiomers, polymorphs, amorphous and crystal forms. Lactose, which is used as carrier, can optionally be added in a higher or a lower amount.

The example can be repeated by replacing the amounts in Example 20 with the amounts given in the table below.

| SAMPLE | Amount of Tiotropium bromide (parts) | Amount of fluticasone propionate (parts) | Amount of lactose (parts) |
|---|---|---|---|
| 21 | 9 | 200 | 11000 |
| 22 | 18 | 200 | 12000 |
| 23 | 18 | 200 | 14000 |
| 24 | 9 | 200 | 15000 |
| 25 | 9 | 200 | 13000 |
| 26 | 18 | 200 | 12500 |
| 27 | 9 | 400 | 15000 |
| 28 | 9 | 400 | 15500 |
| 29 | 18 | 400 | 12000 |
| 30 | 9 | 200 | 12500 |
| 31 | 9 | 200 | 16000 |
| 32 | 18 | 400 | 17000 |
| 33 | 9 | 400 | 16750 |
| 34 | 9 | 400 | 12500 |
| 35 | 18 | 200 | 13500 |
| 36 | 9 | 400 | 14500 |
| 37 | 9 | 400 | 17000 |
| 38 | 18 | 400 | 16500 |

## Claims

1. A medicament composition in dry powder form **characterized in that** said dry powder medicament composition contains tiotropium bromide with water content less than or equal to 2.5% and fluticasone propionate wherein;
• the mean particle size of tiotropium bromide with water content less than or equal to 2.5% and fluticasone propionate is in the range of 1.5 to 4.5 µm and
• said active agents are present in the composition with the ratio of 1:3 to 1:50 respectively and
• lactose which has a mean particle size less than 100 µm is used as carrier stored in capsules or blisters wherein the inside layer of the blister cavity or the capsule material that is in contact with the dry powder formulation is an antistatic material.

2. The medicament composition in dry powder form according to claim 1, wherein tiotropium bromide with water content less than or equal to 2.5% comprises its pharmaceutically acceptable solvates, polymorphs, crystal forms and amorphous forms and/or combinations thereof.

3. The medicament composition in dry powder form according to claim 1, wherein fluticasone propionate comprises its pharmaceutically acceptable solvates, hydrates, enantiomers, racemates, polymorphs, crystal forms and amorphous forms and/or combinations thereof.

4. The medicament composition in dry powder form according to claim 2, wherein the amount of tiotropium bromide with water content less than or equal to 2.5% is in the range of 1 to 40µg.

5. The medicament composition in dry powder form according to claim 3, wherein the amount of fluticasone propionate and/or pharmaceutically acceptable derivatives of its is in the range of 1 to 600 µg.

6. The medicament composition in dry powder form according to claim 1, wherein lactose that has a mean particle size less than 100 µm is used as a mixture of particles having two different mean particle sizes.

7. The medicament composition in dry powder form according to claim 6, wherein lactose that has mean particle size less than 100 µm is present as a mixture of particles having a mean particle size less than 10 µm and particles having a mean particle size in the range of 10 µm to 100 µm.

8. The medicament composition in dry powder form according to claim 7, wherein the fine lactose particles have a mean particle size between 2 µm and 8µm and the coarse lactose particles have a mean particle size between 30 µm and 80 µm.

9. The medicament composition in dry powder form according to claim 7, wherein the ratio of the fine lactose, particles which has a mean particle size less than 10 µm, to the coarse lactose particles, which has a mean particle size in the range of 10 µm to 100 µm, is in the range of 20:80% and 40:60% by weight.

10. The medicament composition in dry powder form according to claim 1, wherein the amount of lactose is preferably in the range of 0,1-50 mg.

11. The medicament composition in dry powder form according to claim 1, wherein the antistatic material used is antistatic itself or which is not antistatic itself but contains antistatic agent(s).

12. The medicament composition in dry powder form according to claim 11, wherein the antistatic agents used are selected from a group comprising long-chain aliphatic amines (optionally ethoxylated) and amides, glycerol monostearate (GMS), saturated fatty acids, (poly)unsaturated fatty acids, quaternary ammonium salts (e.g., behentrimonium chloride or cocamidopropyl betaine), sulfonated organic compounds, esters of phosphoric acid, polyethylene glycol esters, or polyols and combinations thereof.

13. The medicament composition in dry powder form comprising tiotropium bromide with water content less than or equal to 2.5% and fluticasone propionate wherein;
• the mean particle size of tiotropium bromide with water content less than or equal to 2.5% and fluticasone propionate is in the range of 1.5 to 4.5 µm and
• said active agents are present in the composition with the ratio of 1:3 to 1:50 respectively and
• lactose which has mean particle size less than 100 µm is used as carrier and said composition is stored in capsules or blisters wherein the inside layer of the blister cavity or the capsule material that is in contact with the dry powder formulation is an antistatic material for use in treatment of respiratory diseases especially asthma, allergic rhinitis, chronic obstructive pulmonary disease (COPD).

## Patentansprüche

1. Medikamentenzusammensetzung in trockener Pulverform, **dadurch gekennzeichnet, dass** das Trockenpulvermedikament-Zusammensetzung Tiotropiumbromid mit einem Wassergehalt weniger als oder gleich zu 2.5% und Fluticasonpropionat enthält, wobei;
• die mittlere Teilchengröße von Tiotropiumbromid mit einem Wassergehalt weniger als oder gleich zu 2.5% und Fluticasonpropionat im Bereich von 1,5 bis 4.5 µm liegt und
• die erwähnte Wirkstoffe in der Zusammensetzung mit dem Verhältnis von 1:3 bis 1:50 jeweils vorhanden sind und
• Laktose, die eine mittlere Teilchengröße weniger als 100 µm hat, als Träger verwendet wird, der in Kapseln oder Blistern aufbewahrt wird, wobei die innere Schicht der Blisterkavität oder Kapselmaterial , das mit der Trockenpulverformulierung in Kontakt ist, ein antistatisches Material ist.

2. Medikamentenzusammensetzung in Trockenpulverform nach Anspruch 1, wobei Tiotropiumbromid mit einem Wassergehalt weniger als oder gleich zu 2.5% dessen pharmazeutisch annehmbaren Lösungsmitteln, Polymorphen, Kristallformen und amorphen Formen und / oder Kombinationen davon umfasst.

3. Medikamentenzusammensetzung in Trockenpulverform nach Anspruch 1, worin Fluticasonpropionat dessen pharmazeutisch annehmbaren Solvaten, Hydraten, Enantiomeren, Racematen, Polymorphen, Kristallformen und amorphen Formen und/oder Kombinationen davon umfasst.

4. Medikamentenzusammensetzung in Trockenpulverform nach Anspruch 2, wobei die Menge von Tiotropiumbromid mit einem Wassergehalt weniger als oder gleich zu 2.5% im Bereich von 1 bis 40 µg liegt.

5. Medikamentenzusammensetzung in Trockenpulverform nach Anspruch 3, wobei die Menge von Fluticasonpropionat und/oder seinen pharmazeutisch annehmbaren Derivaten im Bereich von 1 bis 600 µg liegt.

6. Medikamentenzusammensetzung in Trockenpulverform nach Anspruch 1, wobei die Laktose mit einem mittleren Teilchengröße weniger als 100 µm als eine Mischung von Teilchen verwendet wird, die zwei unterschiedlichen mittleren Teilchengrößen aufweisen.

7. Medikamentenzusammensetzung in Trockenpulverform nach Anspruch 6, wobei Laktose, die eine mittlere Teilchengröße weniger als 100 µm hat , als eine Mischung von Partikeln mit einer mittleren Teilchengröße weniger als 10 µm und von Partikeln mit einer mittleren Teilchengröße im Bereich von 10 µm bis 100 µm vorhanden ist.

8. Medikamentenzusammensetzung in Trockenpulverform nach Anspruch 7, wobei die feine Partikel der Laktose eine mittlere Teilchengröße zwischen 2 µm und 8 µm haben und die grobe Partikel der Laktose eine mittlere Teilchengröße zwischen 30 µm und 80 µm haben.

9. Medikamentenzusammensetzung in Trockenpulverform nach Anspruch 7, wobei das Verhältnis der Teilchen der feinen Laktose mit einer mittleren Teilchengröße weniger als 10 µm zu der Teilchen der groben Laktose, die eine mittlere Teilchengröße im Bereich von 10 µm bis 100 µm hat, im Bereich von 20:80% und 40:60% nach Gewicht liegt.

10. Medikamentenzusammensetzung in Trockenpulverform nach Anspruch 1, wobei die Menge der Lactose vorzugsweise im Bereich von 0,1-50 mg liegt.

11. Medikamentenzusammensetzung in Trockenpulverform nach Anspruch 1, wobei das verwendete antistatische Material antistatisch oder nicht antistatisch selbst ist, sondern enthält Antistatikum (Antistatika).

12. Medikamentenzusammensetzung in Trockenpulverform nach Anspruch 11, wobei die verwendeten antistatischen Mittel aus einer Gruppe ausgewählt werden, die langkettige aliphatische Amine (gegebenenfalls ethoxyliert) und Amide, Glycerinmonostearat (GMS), gesättigte Fettsäuren, (poly) ungesättigte Fettsäuren, quartäre Ammoniumsalze (beispielsweise Behentrimoniumchlorid oder Cocamidopropylbetain), sulfonierte organische Verbindungen, Ester von Phosphorsäure, Polyethylenglykolester oder Polyole und Kombinationen davon umfasst.

13. Medikamentenzusammensetzung in Trockenpulverform, enthaltend Tiotropiumbromid mit einem Wassergehalt weniger als oder gleich zu 2.5% und Fluticasonpropionat wobei;
• die mittlere Teilchengröße von Tiotropiumbromid mit einem Wassergehalt weniger als oder gleich zu 2.5% und Fluticasonpropionat im Bereich von 1,5 bis 4.5 µm liegt und
• die genannten Wirkstoffe in der Zusammensetzung mit dem Verhältnis von 1:3 bis 1:50 jeweils vorhanden sind und
• Laktose, die eine mittlere Teilchengröße weniger als 100 µm hat als Träger verwendet wird und die genannte Zusammensetzung in Kapseln oder Blistern aufbewahrt wird, wobei die innere Schicht der Blisterkavität oder Kapselmaterial, das in Kontakt mit der trockenen Pulverformulierung ist, ein antistatisches Material zur Verwendung bei der Behandlung von Atemwegserkrankungen insbesondere wie Asthma, allergische Rhinitis, chronisch-obstruktive Lungenerkrankung (COPD) ist.

## Revendications

1. La compositison medicamenteuse sous forme de poudre sèche caractérisée ladite composition de médicament en poudre sèche contient du bromure de tiotropium avec une teneur en eau inférieure ou égale à 2.5% et du propionate de fluticasone dans laquelle;
• la dimension moyenne partide de bromure de tiotropium avec une teneur en eau inférieure ou égale à 2.5% et du propionate de fluticasone est dans la gamme de 1.5 à 4.5 µm et
• lesdits agents actifs sont présents dans la composition avec une intervalle de 1: 3 à 1:50 respectivement et
• Le lactose qui a une dimension moyenne de partide inférieure à 100 µm est utilisé comme support stocké dans des capsules ou blisters dans lequel la couche intérieure de la cavité de blister ou le matériau de la capsule qui est en contact avec la formulation de poudre sèche est un matériau antistatique.

2. La compositison medicamenteuse sous forme de poudre sèche selon la revendication 1, dans laquelle le bromure de tiotropium avec la teneur en eau inférieure ou égale à 2.5% comprend sa pharmaceutiquement acceptables solvantes polymorphes, formes cristallines et formes amorphes et/ou des combinaisons de ceux-ci.

3. La compositison medicamenteuse sous forme de poudre sèche selon la revendication 1, dans laquelle propionate de fluticasone comprend sa pharmaceutiquement acceptables solvantes hydrates, énantiomères, racémates, polymorphes, formes cristallines et amorphes formes et/ou des combinaisons de ceux-ci.

4. La compositison medicamenteuse sous forme de poudre sèche selon la revendication 2, dans laquelle la quantité de bromure de tiotropium avec la teneur en eau inférieure ou égale à 2.5% est dans l'intervalle de 1 à 40 µg.

5. La compositison medicamenteuse sous forme de poudre sèche selon la revendication 3, dans laquelle la quantité de propionate de fluticasone et/ou les pharmaceutiquement acceptable dérivés son dans l'intervalle de 1 à 600 µg.

6. La compositison medicamenteuse sous forme de poudre sèche selon la revendication 1, dans laquelle le lactose qui a une dimension moyenne de particules inférieure à 100 µm est utilisé comme un mélange de particules ayant deux differentes dimensions moyennes de particules.

7. La compositison medicamenteuse sous forme de poudre sèche selon la revendication 6, dans laquelle le lactose qui a une dimension moyenne inférieure à 100 µm est présentée ayant une dimension moyenne de particules inférieure à 10 µm et ayant une dimension moyenne dans l'intervalle de 10 µm à 100 µm.

8. La compositison medicamenteuse sous forme de poudre sèche selon la revendication 7, dans laquelle le lactose fine particules ont une dimension moyenne de particules dans l'intervalle de 2 µm et 8 µm et les particules de lactose coarse ont une dimension moyenne de particules dans l'intervalle de 30 µm et 80 µm.

9. La compositison medicamenteuse sous forme de poudre sèche selon la revendication 7, où la ratio du lactose fine, de particules qui ont une dimension moyenne de particules inferieure à 10 µm aux particules lactose qui ont une dimension moyenne de particules dans l'intervalle de 10 µm à 100 µm, est dans l'intervalle de 20:80% et 40:60% par poids.

10. La compositison medicamenteuse sous forme de poudre sèche selon la revendication 1, dans laquelle la quantité de lactose est de préférence dans l'intervalle de 0,1-50 mg.

11. La compositison medicamenteuse sous forme de poudre sèche selon la revendication 1, dans laquelle le matériau antistatique utilisé en lui-même antistatique ou qui n'est pas lui-même antistatique mais qui contient un agent (s) antistatique.

12. La compositison medicamenteuse sous forme de poudre sèche selon la revendication 11, dans laquelle les agents antistatiques sont choisis parmi un groupe composé à longue chaîne, les amines aliphatiques (éventuellement éthoxylés) et les amides, le monostéarate de glycerol (GMS), des acides gras saturés, les (poly) gras insaturé les acides, les sels d'ammonium quaternaire (par exemple, le chlorure de behentrimonium ou cocamidopropylbétaïne), les composés organiques sulfonés, les esters d'acide phosphorique, les esters de polyéthylène glycol ou de polyols et leurs combinaisons.

13. La composition de médicament sous forme de poudre sèche comprenant du bromure de tiotropium avec une teneur inférieure ou égale à 2,5% et du propionate de fluticasone dans laquelle l'eau;
• la dimension moyenne des particules de bromure de tiotropium avec une teneur en eau inférieure ou égale à 2.5% et du propionate de fluticasone est dans la gamme de 1.5 à 4.5 µm et
• lesdits agents actifs sont présents dans la composition avec un rapport de 1: 3 à 1:50, respectivement, et
• le lactose qui a une dimension moyenne de particules inférieure à 100 µm est utilisée comme support et ladite composition est stockée dans des capsules ou ampoules dans laquelle la couche intérieure de la cavité de blister ou le matériau de la capsule qui est en contact avec la formulation de poudre sèche est un matériau antistatique pour utiliser dans le traitement des maladies respiratoires, en particulier l'asthme, la rhinite allergique, bronchopneumopathie chronique obstructive (BPCO).
